# EUROPEAN PATENT SPECIFICATION

(11) **EP 0 951 646 B1**
(45) Date of publication and mention of the grant of the patent: **23.06.2004**
(21) Application number: 97953598.6
(22) Date of filing: 19.12.1997
(51) Int. Cl.: G01N 33/543, G01N 33/541, G01N 33/535, G01N 33/533, G01N 33/566, G01N 33/569

(54) **THE IMMUNOENZIMATIC ASSAY FOR THE DIAGNOSIS OF EQUINE INFECTIOUS ANEMIA VIRUS DISEASE BY USING RECOMBINANT PROTEIN (rGP90) DERIVED FROM EQUINE INFECTIOUS ANEMIA VIRUS**
IMMUNOENZYMATISCHER ASSAY FÜR DIE DIAGNOSE VON INFEKTIÖSER PFERDEANÄMIE-VIREN-KRANKHEIT MIT HILFE DES REKOMBINANTEN VIRUSPROTEINS RGP90
DOSAGE IMMUNO-ENZYMATIQUE POUR DIAGNOSTIQUER L'ANEMIE INFECTIEUSE EQUINE AU MOYEN DE LA PROTEINE RECOMBINANTE (gp90) DERIVEE DU VIRUS DE L'ANEMIE INFECTIEUSE EQUINE

(30) Priority: 18.12.1996 BR 9600062
(43) Date of publication of application: 27.10.1999
(73) Proprietor: Universidade Federal De Minas Gerais, CEP-31270-901 Belo Horizonte, MG (BR)
(72) Inventor: PEREGRINO FERREIRA, P.,César Apart.201, CEP-31275-060 Belo Horizonte, MG (BR); KROON, Erna, Geessien, CEP-31365-640 Belo Horizonte, MG (BR); PIMENTA DOS REIS, Jenner, Karlisson, CEP-31760-100 Belo Horizonte, MG (BR); FORTES FERRAZ, Isabella, Bias, CEP-30320-480 Belo Horizonte, MG (BR); CERQUEIRA LEITE, Rômulo, CEP-31330-090 Belo Horizonte, MG (BR)
(74) Representative: Larfeldt, Helene
(86) International application number: PCT/BR1997/000083
(87) International publication number: WO 1998/027428

(56) References cited:
- US-A- 3 932 601
- CHEMICAL ABSTRACTS, Vol. 124, No. 13, 25 March 1996, (Columbus, Ohio, USA), page 993, Abstract No. 172919b, GRUND C.H. et al., "Fine Specificity of Equine Infectious Anemia Virus gp90-Specific Antibodies Associated with Protective and Enhancing Immune Responses in Experimentally Infected and Immunized Ponies"; & J. GEN. VIROL., 1996, 77(3), 435-42 (Eng).
- CHEMICAL ABSTRACTS, Vol. 116, No. 11, 16 March 1992, (Columbus, Ohio, USA), page 559, Abstract No. 103966n, BALL J.M. et al., "Detailed Mapping of the Antigenicity of the Surface Unit Glycoprotein of Equine Infections Anemia Virus by Using Synthetic Peptide Strategies"; & J. VIROL., 1992, 66(2), 732-42 (Eng).
- CHEMICAL ABSTRACTS, Vol. 108, No. 19, 09 May 1988, (Columbus, Ohio, USA), page 503, Abstract No. 165830g, O'ROURKE K. et al., "Antiviral, Anti-Glycoprotein and Neutralizing Antibodies in Foals with Equine Infectious Anemia Virus"; & J. GEN. VIROL., 1988, 69(3), 667-74 (Eng).

## Description

The present invention relates to a method of detecting antibodies against envelope surface antigen of equine infectious anemia virus (EIAV), using as antigen the non glycosylated recombinant protein (rgp90) in immunoenzymatic assays. More particularly, it relates to the use of non-glycosylated recombinant protein gp 90 in kits for diagnosis of equine infectious anemia (EIA).

### BACKGROUND OF THE INVENTION

The equine infectious anemia (EIA) is one of the oldest diseases caused by virus, having been described for the first time in France by LlGNEE (Rec. Med. Vet, 20:30, 1843) and recognized as viral disease by VALLEE and CARRE (Acad. Sci. ,139:331-333,1904). The disease affects exclusively the members of the family **Equidae** presenting a worldwide distribution and of great economical importance consequently.

The EIA virus (EIAV) is classified as a lentivirus of the Retroviridae family (CHARMAN et al. **J. Virol.** 19(2):1073 -1076,1976), it is genetic and antigenically related to the other lentiviruses which are characterized by developing persistent infection in host. The EIA has played a specially important role in comparative virology and in the studies of the acquired immunodeficiency syndrome (AIDS). Besides their morphological identity, both viruses are similar in terms of nucleotide sequences that code for structural surface proteins. These group of virus present genetic and antigenic variants during persistent infections, which is associated to immune response scape (MONTAGNIER et al. **Ann. Virol.**, 135:119-134, 1984, **MONTELARO** et al. J. **Biol. Chem.**, 259:10539-10544,1984, RUSHLOW et al. **Virology**, 155:309-321, 1986, STREICHER et al. **J. Am. Med. Assoc.** 256:2390-2391, 1986, STOLER et al. **J. Am. Med. Assoc.** 256:2360-2364,1986 and HAHN et al. **Science,** 232:1548-1553, 1986).

The transmission of EIAV occurs mainly through bite of arthropods vectors (tabanideos) which, inoculate the virus into the animal's blood stream (mechanical transmission) when feeding themselves. The way of transmition is responsible for the high prevalence of EIA in areas favorable to the life cycle of vectors (ISSEL et al. **Vet.** 17:251-286, 1988). The EIAV can also be transmitted by the placenta and colostro of mares with high virus levels, and by needles and surgical instruments contaminated with blood (COGGINS **Comparative diagnosis of viral diseases,NY**, 4:646-658, 1981). The course of infections shows different clinical forms of the disease (subacute, chronic and mainly inaparent or assymptomatic) in horses (JSSEL & COGGINS, **J. Am. Vet. Med. Assoc.** 174(7):727-33, 1979) and the most prominent signs are the fever episodes, hemolytic anemia, anorexia, fast weight loss and ventral edema.

The laboratory diagnosis plays a decisive role in the control and prevention of EiA if considering the high prevalence of assymptomatic carriers, non conclusive and possibility to confuse clinical diagnosis with other diseases as the trypanosomiasis, pyroplasmosis, leptospirosis, hepatitis and by parasites.

The diagnose of EIAV has been done through the detection of specific antibodies against surface antigen of virus present in the serum of affected animals by using the Coggins or agar gel diffusion test (U.S.Pat. nro.3,929,982 and U.S.Pat. No. 3,932,601). In the Coggins test the antigen and sample serum is placed side by side in an agarose gel plate. If EIA antibodies are present in the test serum, they will form a precipitin line when diffusing toward the agarose gel .

This methodology is inherently insensitive since EIAV antigen preparation derived from spleen of infected animals or equine derme cultures cells may be contaminated with non-EIAV antigens during its preparation. Besides, antibodies against non-EIAV antigens may be present in the test serum and can react with the non-EIA antigens forming a variety of nonspecific precipitin lines. Even if, EIAV-antigen batches can be purified the Coggins test is laborious, time-consuming and demanding of considerable expertise in interpretation of results. The Coggins test procedure takes twenty-four to forty-eight hours for the formation of clearly visible precipiting lines, delaying results.

The method described by O'Rourke et al. (J. Gen. Virol., Vol. 69, pp 667-674, 1988) improved the diagnosis of EIAV by using an. ELISA assay with purified virus bound to immulon I plates.

Porter (U.S. Pat. No.4,806,467) discloses a method for detecting the EIA virus using a competitite enzyme-linked immunoabsorbent assay incorporating a purified. viral antigen and a monoclonal antibody. To obtain the antigen, the EIAV must first be cultured. The antigen used was p26 core protein of the EIAV and is obtained through (purification of the cultured virus by a variety of means) well known in the art. The technique of virus tissue cultures increases the possibility of assay yield false positive results since the virus may be contaminated with other forms of protein or even another virus. Additionally, the EIAV is hard to culture, making Porter's approach very difficult for large scale production.

The use of a synthetic peptide in an enzyme linked immunosorbent assay for the detection of human immunodeficiency virus (HIV) was disclosed by Shoeman, R.L et al (Analytical Biochemistry 161:370-379,1987).

HIV and the EIA virus are members of the retrovirus family and have similar structures but distinct amino acid sequences. Ball et al. (J. Virol. Vol. 66, pp 732-742, 1992) described the use of synthetic peptides for mapping antigenicity of gp90 to provide potential peptide substrates for EIAV diagnostic assays. The peptides showed different reactivity towards monoclonal antibodies depending on their length. They also showed different reactivity towards different horse sera, the reactivity ranging from 10% to 100%. Grund et al. (J.Virol. Vol. 77, pp 435-442, 1996) observed the same problem when using synthetic peptide ELISA to characterise the specificity of antibodies to linear determinants of the EIAV surface glycoprotein gp90.

Darrel & Peisheng, the U.S. Patent No. 5,427,907, discloses a method to use a synthetic peptide as the antigen in an immunoassay for the detection of antibodies against the equine infectious anemia virus in the serum of horses. This procedure include only the search of some epitopes of a virus proteins.

It is an object of the present invention to provide an assay for the detection of the equine infectious anemia virus antibodies which may be fast, easily and quickly performed by using the stable non-glycosylated recombinant envelope protein (rgp90) which may be produced in sufficient amounts at a low cost.

### BRIEF DESCRIPTION OF THE DRAWINGS

These and other objects, features and many attendant advantages of the invention will be better understood upon a reading of the following detailed description when considered in connection with the accompanying drawings wherein:
Figure 1 shows schematically the method of diagnosis
Figure 2 shows the titration of positive and negative sera in Elisa with the non-glycosylated recombinant protein rgp90 as antigen.
Figure 3 demonstrates the distribution of the optical density (OD) in Elisa with the recombinant protein gp90 as antigen with 84 positive and 70 negative horses samples, previously tesyed by IDGA and ELISA by using EIAV-antigen produced in cell cultures
Figure 4 demonstrates the optical density of the ELISA reaction with the non-glycosylated recombinant protein gp90 as antigen after EIAV "Wyoming" strain experimental infected-horse.

### DETAILED DESCRIPTION OF THE INVENTION

It is, therefore , an object of the present invention to provide a method of immunodiagnosis for EIA disease that uses the non-glycosylated recombinant protein gp90 corresponding derived from viral envelope of EIAV. The method consists of binding the recombinant antigen to solid supports (microtiter plates, tubes, beads or nitrocelullose or nylon papers or any kind that allow protein binding) and to proceed the analysis of the sera (presence of antibodies) from animals suspected of infection with the EIAV.

The non-glycosylated recombinant protein gp90 is added to a solid phase support and incubated for sufficient time to ensure that protein was bound to the support. The equine test sample is added to the support and incubated for a period of time sufficient to permit that any EIA-antibodies are removed from sample.

Labeled conjugate is added which binds to the protein-antibody complex Following enough time to allow such binding, any unbound labeled conjugate is removed by washing. Labeled conjugate is added wich binds to the protein-antibody complex Following enough time to allow such binding, any unbound labeled conjugate is removed by washing. High level of bound conjugate indicates a positive result, . which mean presence of EIA viral antibodies. A low level of bound conjugate indicates a negative result which mean ausence or undetectable level of EIA viral antibodies..

A variety of commercially available solid phase supports may be used for protein binding. The direct binding of equine antibodies present in the test serum to the solid phase support is likely to result in a false positive reading. To prevent such binding, the blocking solution is used to fill any empty binding sites on the support which did not bind antibodie-protein. Any substance which will not react with EIA viral antibodies and antigen will function as a blocker. A conjugate is something which will recognize and bind with the test serum EIA viral antibody.

The conjugate may be labeled using a variety of labeling means, including but not limited to: enzyme labeling, fluorescent labeling, and magnetic labeling. If enzymatic labeling is the labeling means chosen, the conjugate is labeled with an enzyme preferably select from the group consisting of horseradish peroxidase and alkaline phosphatase. Other enzymes may be used.

When an enzyme label is used, the labeled conjugate is detected by adding an amount of a substrate which will recognize and react with the enzyme label to form a product that will produce a color change visible to the naked eye. The presence of color indicates a sufficient level of test serum antibodies to indicate infection. An absence of color is an indicator of a lack of infection, as the animal did not produce a significant number of antibodies to the virus. Hence, the labeled conjugate had few antibodies, if any, to bind with and was subsequently removed from the support. There are a variety of both peroxidase and phosphatase substrates which will react with horseradish peroxidase and alkalinie phosphatase enzymes, respectively to form a colored product.

A preferred peroxidase substrate is an orthophenylenediamine/hydrogen peroxide solution. The intensity of the color of the product may be quantified using a spectrophotometer to read absorvance. Howewer, measuring the absorvance is not necessary to obtain an accurate reading of the results of the assay.

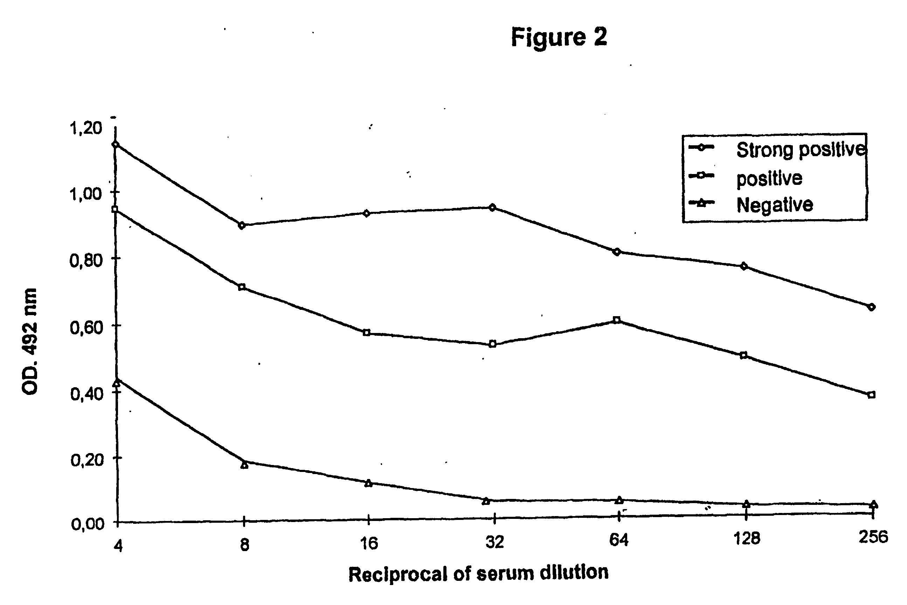

The titration of positive and negative sera in Elisa with 1 µg recombinant protein gp90 as antigen (Figure 2) shows the detection of antibodies anti-gp90 in the ELISA test using dilutions of the serum from 1:4 to 1:256 and obtaining OD from 0.800 to 0.400. The negative controls demonstrate that there is a non specific reaction.

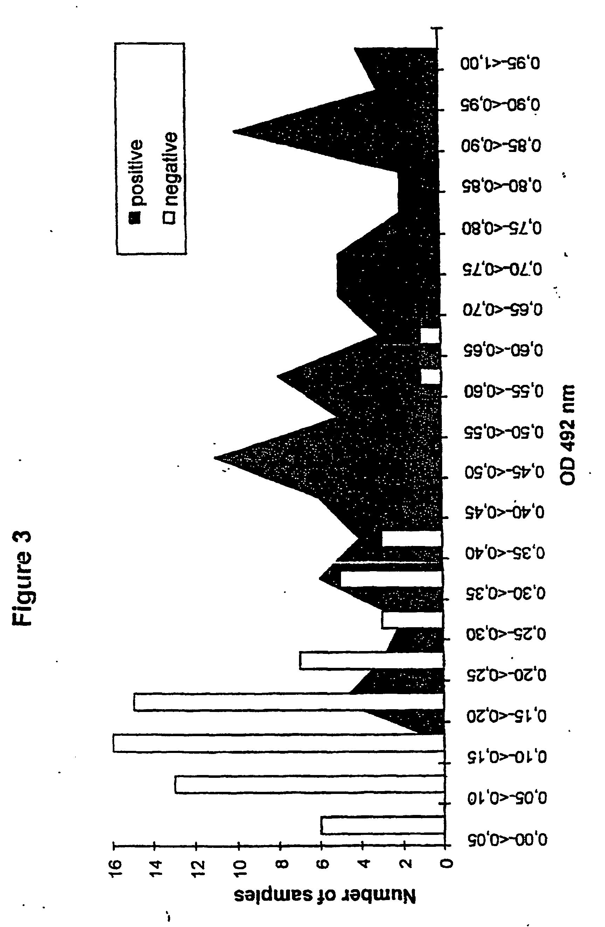

The optical density obtained when sera from 84 positive and 70 negative horses were tested is presented on Figure 3, showing the frequency of the different optical densities obtained.

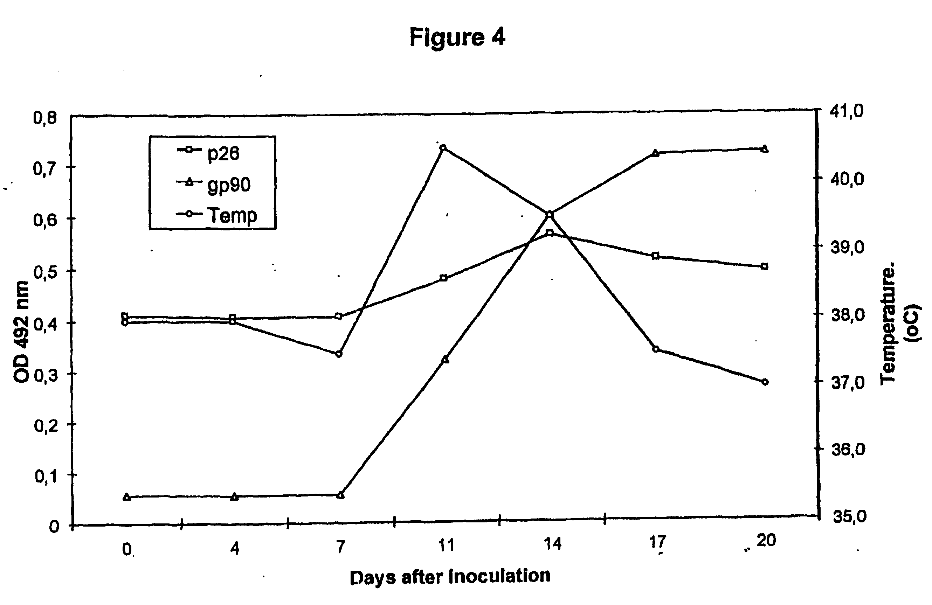

An animal was experimentally infected and its sera tested with the ELISA rgp90. Figure 4 shows that specific antibodies were detected seven days after the infection together with the appereance of fever.

in order that this invention may be better understood the follow examples for illustrative purposes only, are described. The examples illustrate the present invention and are not intended to limit it in spirit or scope.

### EXAMPLE 1

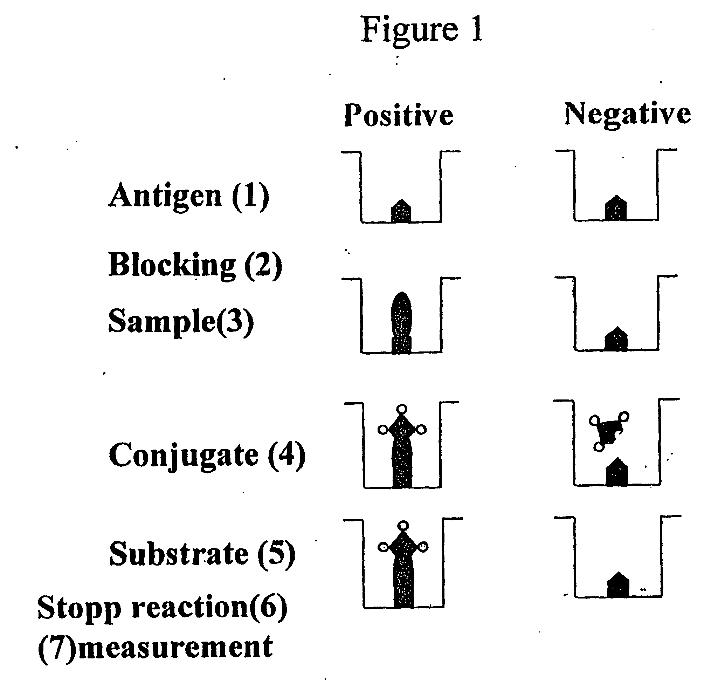

The process can be better understood through the following description in consonance with the illustration in Figure 1 where the binding of the antigen in (non-glycosylated recombinant protein gp90) to the solid support (1), it is done by its dilution in carbonate buffer (Na₂CO3 0.1-0.5M; NaHCO3 0.1-0.5M, pH 8.0-9.6), added in the concentrations of 0.01-1g and incubated the temperature of 4-8°C for 18-24 hours in micro-technique plates, tubes or beads followed by electrotransference or passive transference to nitrocelullose or nylon supports. After antigen binding, the support was washed for 3 to 6 times with buffer solution (0.01-0.02 M NaH₂PO4 , 0.01-0.02 MNa₂HPO4 , 0.02-0.04M KCl, 0.85-0.9% NaCl pH 7.0-7.5),and then with 0.05-0.1% of tween-20 (Buffer-Tween). To block the inespecific sites of binding (2) the used support was incubated with block solution (skimmed powdered milk 1-5% bovine , 1-5%albumin or 1-5% casein in Tween buffer) for 30-60 min at 23°C-37°C. After a new wash of the support with Tween buffer, as described previously, the positive and negative control and the serum samples were diluted in Tween buffer, to bound to the antigen linked to the solid support (3), and incubated at 23°C-37°C. After new wash of the support with Tween buffer, the conjugate was added, where the anti-equine immunoglobuline binds to the antibodies that are tied up to the antigens (4). Conjugate can be an equine anti-immunoglobuline conjugated to the enzyme peroxidase or any other enzyme as acetylcolinesterase, lactate desidrogenase, galactosidase, glicose oxidase, alkaline fosfatase, or another. This conjugate was diluted in Tween buffer in agreement with its title and added to the support and then incubated at 23°C-37°C for 30-60 min. A new wash of the support with Tween buffer and the development of the reaction was proceeded (5) with the enzyme of the conjugate, transforms the substrate of colorless to a red-faced product. The developing solution is composed of the substrate of the enzyme used in the conjugate that for the peroxidase for example is the ortofenilenodiamino diluted in phosphate or citrate buffer 0.1-0.2 M, pH 5.0-8.0. After the color development, which is proportional to the concentration of specific antibodies in each sample, solution of acid was used (sulfuric acid) for stop-reaction (6), where the acid interrupts the previous reaction. For the final result the measurement (7) of the color intensity formed in each reaction (sample) was made. This reading was made visually or in espectrophotometer, in absorbance, with a specific filter for the color formed by the developing solution.

### EXAMPLE 2

The kit for diagnosis of the EIAV may contain the the folowing products: (a) the antigen recombinant gp90 from EIA coated to the solid support (microplate, microtiter wells, tubes, capillary tubes, sticks, dipsticks, beads) with different chemical composition (polystyrene, polypropilene, polyethylene, polypropylene, poly-carbonate, polyvinyl, polystyrene, latex, nitrocellulose, nylon; cellulose, polyacrylamide, cxoss-linked dextran and microcrystalline glass (b) the anti-equine immunoglobulin conjugated with label that is selected from the group consisting of an enzyme, a fluorescent marker, avidin-biotin (c) the substrate for the label as orthophenilenodiamine and H₂O₂ (d) a blocking solution (0.01-0.02M NaH₂PO4, 0.01-0.02M Na₂HPO₄, 0.02-0.04M·KCl, 0,85-0,9% NaCl pH 7.0-7.5), with 0.05-0.1% of Tween 20 and skimmed powdered milk 1-5% bovine, 1-5% albumin or 1-5%casein (e) a diluent solution for specimen and conjugate (0.01-0:02 M NaH₂PO4 ,·0.01-0.02M Na₂HPO4, 0.02-0.04M KCl, 0.85-0.9% NaCl pH 7.0-7.5), with 0.05-0.1% of Tween 20 and 1% skimmed powdered milk (f) a diluent solution for substrate 0.1 M Na₂HPO4, 0.1M C6H8O7 pH 5,0 (f) stop solution 7N H₂SO4 (g) wash solution (0.01-0.02M NaH₂PO4, 0.01-0.02M Na₂HPO4, 0.02-0.04 M KCl, 0.85-0.9% NaCl pH 7.0-7.5), with 0.05-0,1% of Tween 20 (h) positive control inactivated horse serum (I) negative control inactivated horse serum

## Claims

1. An immunoenzymatic assay for detection of antibody by using the equine infectious anemia virus non-glycosylated recombinant gp90 envelope antigen in animal test samples comprising:
(a) binding of the non-glycosylated recombinant gp90 envelope antigen to a solid support,
(b) reacting the bound antigen with a test sample of serum,
(c) removing the unbound test sample,
(d) reacting the bound test antibody with a labeled antibody
(e) measuring the amount of bound antibody specific to the equine anemia infectious virus gp 90 envelope antigen in the test sample

2. The immunoassay according to claim 1, wherein said label is selected from the group consisting of an enzyme, a fluorescent marker, avidin-biotin.

3. The immunoassay according to claim 1, wherein said solid support is selected from the group consisting of polystyrene or polypropilene microtiter wells, polyethylene, polypropylene, polycarbonate, polyvinyl, polystyrene, or glass test tubes, capillary tubes, dipsticks, or beads; latex beads; nitrocellulose, nylon; cellulose, polyacrylamide, cross-linked dextran and microcrystalline glass.

## Patentansprüche

1. Eine immunoenzymatische Analyse zur Entdeckung von Antikörpern unter der Verwendung des nicht-glykosylierten rekombinanten gp90 Hüllenantigens des infektiösen Pferdeanämievirus in einem Tierversuchssample bestehend aus:
a) einer Verbindung des nicht-glykosylierten rekombinanten gp90 Hüllenantigens zu einer festen Stütze;
b) einer Reaktion des gebundenen Antigens mit einem Serumversuchssample;
c) einer Entfernung des nicht-verbundenen Versuchssamples;
d) einer Reaktion des gebundenen Testantikörpers mit einem markierten Antikörper;
e) einer Messung der Menge der gebundenen Antikörper, typisch für das Hüllenantigen gp90 des infektiösen Pferdeanämievirus, in dem Versuchssample.

2. Die immunoenzymatische Analyse nach Anspruch 1, in dem der genannte markierten Antikörper aus der Gruppe eines Enzyms, eine fluoreszierende Markierung, Avidin-Biotin ausgewählt ist.

3. Die immunoenzymatische Analyse nach-Anspruch 1, in dem die genannte feste Stütze aus der Gruppe bestehend aus Polystyren oder Polypropylen Mikrotiter Quellen, Polyäthylen, Polypropylen, Polykarbonat, Polyvinyl, Polystyren, oder Teströhrchen aus Glas, Kapillarröhrchen, Messstäben, oder Tropfen; Latextropfen; Nitrozellulose, Nylon; Zellulose, Polyakrylamid, querverbundenes Dextran und Mikrokristallinglas.

## Revendications

1. Une analyse immunoenzymatique visant la détection d'anticorps en employant le virus équin d'anémie d'infections l'antigène de recombinaison non-glycosylé de l'enveloppe gp90 dans des échantillons relatifs à des tests sur des animaux:
a) une liaison de l'antigène de recombinaison non-glycosylé de l'enveloppe gp90 à un appui plein,
b) réaction de l'antigène lié avec un échantillon d'essai de sérum;
c) enlèvement de l'échantillon non lié à l'essai;
d) réaction de l'anticorps en relation avec l'essai avec un anticorps marqué,
e) mesure de la quantité d'anticorps de détail lié à l'antigène infectieux d'enveloppe gp90 de virus d'anémie équine dans l'échantillon d'essai.

2. L'analyse immunologique selon la revendication 1, où ladite marqué est choisie dans le groupe d'une enzyme, un marqueur fluorescent, avidine-biotine.

3. L'analyse immunologique selon la revendication 1, où ledit appui plein est choisi dans le groupe consistant en polystyrène ou le micro-titre de polypropylène jaillit, polyéthylène, polypropylène, polycarbonate, polyvinyle, tubes de polystyrène et ou en verre à essai, tubes capillaires, jaugeurs, ou goûtes; goûtes de latex, nitrocellulose, nylon, cellulose, polyacrylamide; avec dextrane (polysaccharide composé) et une lame microcrystalline.
